# EUROPEAN PATENT APPLICATION

(11) **EP 3 369 423 A1**
(43) Date of publication of application: **05.09.2018**
(21) Application number: 17305221.8
(22) Date of filing: 01.03.2017
(51) Int. Cl.: A61K 31/715, A61K 31/733, A61K 35/741

(54) **SYNBIOTIC COMPOSITION AND ITS USE FOR PREVENTING AND/OR TREATING NEURODEGENERATIVE DISORDERS**

(71) Applicant: REMINISCIENCES, 75007 Paris (FR)
(72) Inventor: The designation of the inventor has not yet been filed
(74) Representative: Santarelli

(57) **Abstract**

The invention relates to a new synbiotic composition comprising a mixture of probiotic and prebiotic ingredients for the prevention and/or treatment of neurodegenerative disorder, in particular Alzheimer's disease. The new synbiotic composition can comprise, in particular, at least one *Roseburia* species and at least one *Verrucomicrobiales* species in combination with dietary fibers.

## Description

### FIELD OF THE INVENTION

The invention relates to a new synbiotic composition for use in preventing development of and/or treating neurodegenerative disorders. More particularly, the present invention relates to a synbiotic composition comprising a specific combination of bacterial strains and fibers which can be used in the treatment and/or in the prevention of neurodegenerative disorders, in particular Alzheimer's disease.

### BACKGROUND OF THE INVENTION

Food companies have increased their activities to identify food products which will provide beneficial health effects on the consumer in addition to providing essential nutrients named functional foods. Examples are viable microorganisms, so called probiotics which have shown specific health benefits on gut health and which are included in food product. Other food products with health benefits are foods containing dietary fibers and/or prebiotic carbohydrates.

Ageing is associated with impaired cognitive function. Most aging individuals die from atherosclerosis, cancer, or dementia; but in the oldest old, loss of muscle strength resulting in frailty is the limiting factor for an individual's chances of living an independent life until death.

Alzheimer's disease is a devastating neurodegenerative disorder that impairs patients' memory with disastrous socio-economic consequences. It affects 60 million of the world population above the age of 65 years and, as life expectancy increase, is expected to affect 100 million by 2025. It is neuropathologically characterized by the formation of insoluble protein aggregates - or amyloids - that accumulate in Alzheimer patient's brains. The presence of amyloids in the Alzheimer brain is an early and critical event that leads to the death of neurons and eventually dementia. Thus, arguing in favor of this hypothesis, the most rational strategy for an AD therapy would be to retard Aβ aggregation. To date, there is no cure for this devastating neurodegenerative disorder. Both clinical and epidemiological evidence suggest that, among other non-genetic factors, modification of lifestyle factors such as nutrition may be crucial to the development of AD neuropathology. One potential target against non-healthy nutrition is the gut microflora community or microbiota. Diet is a well-characterized modulator of intestinal microflora. However, daily meal ingestion constitutes an uncontrolled way to modulate microflora. Much of the evidence regarding the role of fermentable gut substrates on host metabolism stem from studies with inulin showing benefits on glucose metabolism and weight regulation in animal experimental models.

The need for preventive strategies is thus urgent. Diet based prevention is recognized as the most efficient strategy to modulate gut microbiota in the combat of life style related disease, and epidemiological studies support that high intake of fibers and legumes is beneficial in the prevention and management of healthy ageing.

The human gastro-intestinal (GI) tract hosts billions of bacteria, which triggers many pleiotropic effects including the immune system homeostasis, food processing, production and secretion of vitamins, modification of bile acid and communication with the Central Nervous System (CNS). Perturbation of the microbial community in the gastro-intestinal tract may contribute to an impaired homeostasis associated with disease states, such as neurological disorders. As aforementioned, both genetic and environmental factors are important in the etiology of AD. A potentially important environmental factor is abnormal intestinal flora that often interacts with other factors such as intestinal permeability and transport of toxic substances.

WO 2012/159023 relates to a method of assessing the presence or the risk of development of hepatic encephalopathy (HE) in a patient with liver disease, comprising a step of analyzing gut microflora in order to determine a gut microbiome signature for said patient. The presence of *Roseburia* in stool samples is indicated as a bacteria associated with better cognition. However, the examples of WO 2012/159023 show that there is decreased abundance of autochtonous *Roseburia* bacteria in HE patient as compared to non-HE patients (example 2, table 11, p.44).

WO2013/176774 relates to microbiome markers and therapies for autism spectrum disorders. Bacteriae of interest belong to *Prevotella*, *Coprococcus*, *Prevotellaceae* or *Veillonellaceae. Verrucomicrobiae* are mentioned on p.12-13 and Table 6, while their different abundance in the gut microflora in neurotypical and autistic groups did not show statiscal significance. Bacteriae of the genus *Roseburia* are cited in Tables 7 and 8 (p. 13-15) while no information is given on their significance. *Akkermansia* is mentioned as one of the top five more abundant genera in both neurotypical and autistic groups (paragraph [0043]) and the prevalence of *Akkermansia* in several autistic subjects was noted, though a correlation between the abundance of *Akkermansia* and the severity of gastrointestinal problems was not found (paragraph [0057]).

WO2015/181449 discloses a method for diagnosis, treatment or prevention of Parkinson's disease comprising determining the relative abundance of one or more microbial taxa, in particular *Prevotelleceae*, in a gut microbiota sample. The abundance of *Verrucomicrobiaceae* was determined and showed that it was associated with the severity of non-motor symptoms and in particular constipation Table 5 and p.22).

The involvement of gut microbiota in the development of Abeta amyloid pathology was shown in a germ-free APP transgenic mouse model (T. Harach et al., 2017,, Scientifc Reports 7, Article number: 41802).

A review of the trend of ongoing research in the field of probiotics and prebiotics is given in Y. Dixit et al., J. Food Microbiol. Saf. Hyg., 2016, 1, 1-13.

### SUMMARY OF THE INVENTION

The invention provides a novel synbiotic composition comprising mixtures of probiotics and prebiotics, as well as their use for the effective prevention and/or treatment of neurodegenerative diseases, in particular Alzheimer's disease, without unwanted side effects. The synbiotic composition comprises a mixture of beneficial microbes (probiotics) and a combination of fibers (prebiotics) suitable for oral ingestion.

The invention relates to the unique finding that bacterial species belonging to the *Roseburia* genus have beneficial effects on host health, in particular when associated to bacterial species belonging to the *Verrucomicrobiales* family. The species belonging to *Roseburia* and *Verrucomicrobiales* can be used in a synbiotic compsition, as a probiotic, together with dietary fibers, as a prebiotic. This association makes it possible to ensure the bacterial viability and benefical activity on the host gut microbiota, by providing the bacteriae with substrates that stimulate their growth and/or activity.

The invention thus relates to a synbiotic product composition comprising a probiotic composition, which comprises at least one *Roseburia* species, preferably at least one *Roseburia* species and at least one *Verrucomicrobiales* species, and a prebiotic mixture, which comprises at least one dietary fiber, and to its use for the prevention and/or treatment of neurodegenerative diseases, in particular those related to pathological ageing, such as dementia and Alzheimer's disease. The invention further relates to a pharmaceutical composition containing said symbiotic composition and a pharmaceutically acceptable vehicle.

As even a mild alteration of gut microbiota (GMB) is related to a lower performance in cognitive tests, the present invention describes the concept linking benefits of GMB modulation to neurodegenerative diseases, in particular those related to pathological ageing. Also, the synbiotic composition of the invention provides neuroprotective, as well as reduced beta-amyloid and plaque accumulation, thereby preventing against dementia and Alzheimer's disease.

### DEFINITIONS

**Probiotics :** viable microorganisms which, when administered in sufficient numbers, confer health benefits to the host.

**Prebiotics :** non digestible selectively fermentable ingredients that allow specific changes in the composition and/or activity of the resident microflora by selectively augmenting the probiotic bacteria.

**Synbiotics:** is the term used for a composition which comprises probiotics (non-pathogenic microorganisms) in addition to prebiotics (natural substrate for the aforementioned microorganisms)

**Amyloid**: Amyloids are insoluble fibrillar protein aggregates that share specific structural traits. Amyloids arise from at least 20 misfolded proteins and polypeptides present naturally in the body. These inappropriately folded structures alter their proper configuration such that they erroneously interact with one another or other cell components forming insoluble fibrils. Amyloids have been associated with the pathology of more than 20 serious human diseases in that, abnormal accumulation of amyloid fibrils in organs may lead to amyloidosis, and may play a role in various neurodegenerative disorders

**Microbiota/Microflora:** The term "microbiota or microflora" refers to the whole microbial community found in the gastro-intestinal tract of a higher organism, including bacteria, archaea, yeasts, and various parasites.

**Microflora modulation**: Refers to a change in the representation of bacteria in a microbiological community of a particular individual. Microflora can be modulated by compounds that induce the growth and/or activity of commensal microorganisms that contribute to the well-being of their host. These coumpounds called **prebiotics** are present in diet or in the gastro-intestinal tract and are typically but not exclusively non-digestible fibers compounds that stimulate the growth and/or activity of beneficial bacteria that colonize the large bowel by acting as substrate for them. Microflora can also be modulated by microorganisms. These live micro-organisms called **probiotics**, when administered adequately, confer a health benefit to the host. Gastro-intestinal microflora can be also modulated by **antibiotics.** These molecules refer to any substance produced by a living microorganism which is antagonistic to the growth and/or division of other living microorganisms. Nutritional approach leading to modulation of gut microbiota by a combination of probiotics and prebiotics are commonly referred as **synbiotics.**

**Microbiome analysis:** Techniques for characterizing the microbiome include use of nucleic acid and/or proteins. Nucleic acid analysis includes analysis of, DNA, RNA, mRNA, rRNA, and/or tRNA, and can be accomplished using, but not limited to pyrosequencing, qPCR, RT-qPCR, clone libraries, DGGE, T-RFLP, ARISA, micro arrays, FIFH, dot-blot hybridization, next generation sequencing, DNA mapping devices and any other DNA hybridization methods that will detect a specific sequence.

**Proteome analysis:** Protein analysis can be performed by 2-Dimensional Gel Electrophoresis, 2-Dimensional Difference Gel Electrophoresis (2D-DIGE), MALDI TOFMS, (2D-) LC-ESI-MS/MS, AQUA, and iTRAQ.

These characterizations are combined with statistical analysis (Linear discriminant analysis effect size (LEfSE, www.huttenhower.sph.harvard.edu/galaxy/)) to precisely determine the players within the microbiome. Rigorous bioinformatics analysis provides accurate characteristics and distributions of intestinal microflora between individuals.

### DETAILED DESCRIPTION OF THE INVENTION

The present invention provides a synbiotic composition comprising probiotics and prebiotics, and its use in preventing and/or treating various neurodegenerative disorders, Alzheimer's disease (AD) in particular, as well as pharmaceutical compositions comprising this synbiotic composition.

The synbiotic composition comprising probiotics and prebiotics of the present invention supplies a specific bacterial equilibrium to the gastro-intestinal (GI) tract. This synbiotic composition alters the bacterial profile in the GI tract and makes it possible to prevent or cure neurodegenerative disorders.

The invention thus relates to a synbiotic composition, comprising
i) a probiotic composition, which comprises at least one *Roseburia* species, and
ii) a prebiotic mixture, which comprises at least one dietary fiber.

Preferably, said probiotic composition comprises at least one *Roseburia* species and at least one or more additional microbial species, for example at least one *Verrumicrobiales* species. A preferred species of *Verrumicrobiales* is *Akkermansia muciniphila.*

Preferred species of *Roseburia* include *Roseburia intestinalis, Roseburia cecicola, Roseburia faecis, Roseburia hominis* and *Roseburia inulinivorans.*

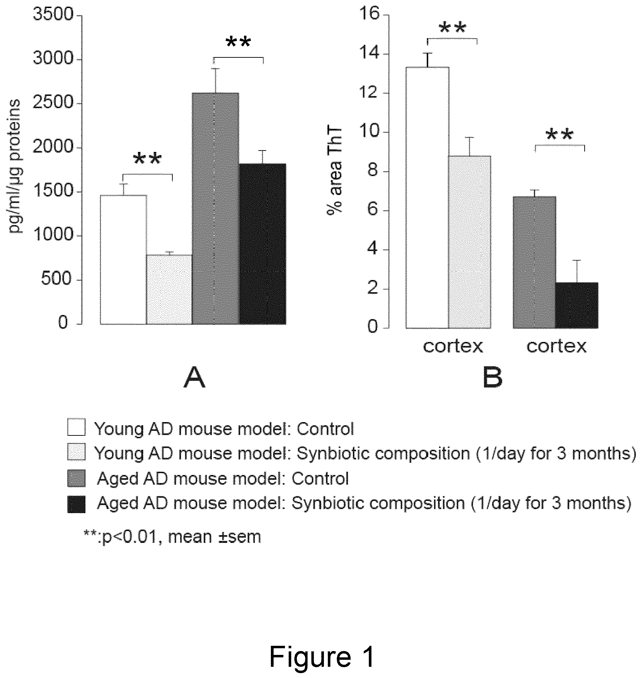

The inventors have now shown that administration of the synbiotic composition comprising probiotics and prebiotics according to the invention into a mouse model of AD leads to a reduction of cerebral levels of soluble Abeta42, which is the most pathogenic Abeta isoform (Figure 1A), as well as cerebral plaques (Figure 1B). Reduction of Abeta 42 levels occurs in both young and aged animals. Furthermore, oral administration of a synbiotic composition comprising prebiotics and probiotics according to the invention induces reduction in cortical Abeta 42 levels.

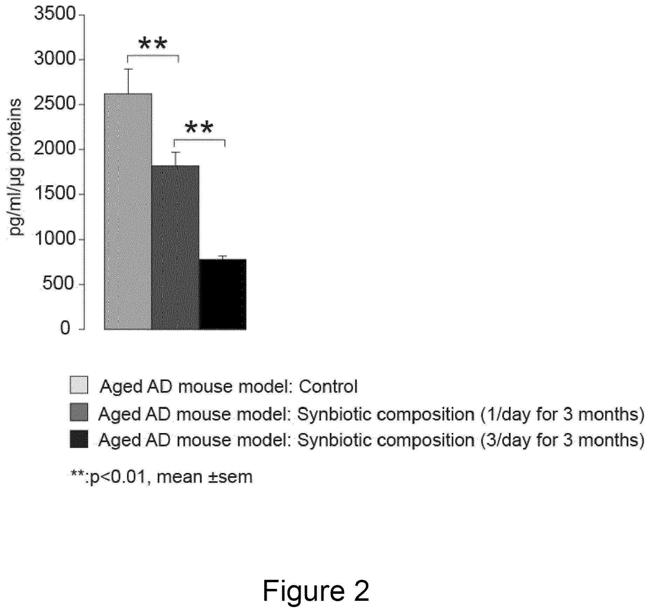

Advantageously, the synbiotic composition of the invention reduces cerebral Abeta 42 in a dose-dependent manner (Figure 2).

Unexpectedly, it has thus been found that the modulation of non-animal elements could make it possible to act on brain pathologies in an animal model.

In particular, it has been found that the composition comprising at least one species of *Roseburia* genus and a *Verrumicrobiale* species, in particular *Akkermansia muciniphila,* in combination with dietary fibers possesses beneficial effects with respect to the treatment and/or prevention of neurodegenerative disorders, in particular Alzheimer's disease.

Affecting the gut microbiota composition by increasing the proportion of at least one *Roseburia* species, and, optionally, at least one *Verrucomicrobiales* species, and providing fibers resulted in increased gut fermentation activity (breath hydrogen (H2) as a test marker), increased concentrations of SCFA and improved behaviour in a mouse model of AD.

The prebiotics which are part of the symbiotic composition of the invention can be selected from dietary fibers, namely carbohydrates including three or more monomers that resist digestion and absorption in the small intestine, and are completely or partially fermented in the colon by gut bacteria. Dietary fibers can be selected from natural or synthetic fibers, purified fibers or mixtures thereof. As understood in the present description, dietary fibers relate to non-starch derived indigestible polysaccharides such as beta-glucans, arabinoxylans, cellulose, oligosaccharides, fructans, pectin, guar gum. Indigestible substrates that are closely associated with indigestible polysaccharides are also included in the definition (e.g., phenolic compounds, lignin and phytosterols).

For example, dietary fibers from cereals or vegetables such as from barley, rye, wheat, oats, vegetable seeds, beta-glucans, guar gum, lignin, lignans and oligosaccharides such as galacto-oligosaccharides and fructo-oligosaccharides, or else chemically modified starch can be used. Examples of resistant starch (RS) are vegetable starches such as retrograded starch, botanically encapsulated starch, starch and cyclodextrins.

According to the invention, dietary fibers can be selected, for example, from β-glucans (such as chitine and cellulose), Hemicelluose (Hexoses, pentoses, lignine, xanthum gum and resistant starch) or else from arabinoxylose, fructans, for example galacto-oligosaccharides (GOS), and inulin, or mixtures thereof.

A combination of galacto-oligosaccharides (GOS) and inulin is preferred.

In particular, the amount of aforementioned prebiotic is between 10% and 50% w/w of the synbiotic composition.

The combination of probiotic (bacteriae) and their substrate (prebiotic) enhances the beneficial impact of the microorganisms on health as the included substrate (fibers) will continuously provide an energy source. Consequently, in combination with fibers contained in ingested meals, fibers of the synbiotic composition of the invention serves as an energy booster for exogenous and endogenous fermenters.

The single or combined use of the symbiotic composition of the invention, in particular containing *Roseburia intestinalis* and *Akkermansia muciniphila*, can also, for example be provided as dry powder and packed in disposable containers to be added to meals, e.g. breakfast cereals.

Preferably, the invention provides a synbiotic composition in a dosage form containing 10⁶ to 10¹¹ viable colony forming units (CFU) per dose. In yet another embodiment, the synbiotic composition of the invention comprises less than 60% probiotics on a weight-percentage basis. In another embodiment, the synbiotic composition comprises at least 20%, in particular 40% of the aforementioned probiotics on a weight-percentage basis.

If more than one bacterial species is present, the species may be present in differing amounts or equal amounts, on a weight percentage basis, with respect to each other. For example, a synbiotic composition according to the invention can comprise 50% probiotic on a weight percentage basis, i.e., a 500 mg dose of a synbiotic composition according to the invention can comprise 250 mg of a probiotic composition. The probiotic composition, in turn, can consist of 30wt% *Roseburia intestinalis,* 20wt% *Roseburia cecicola*, and 50wt% *Akkermansia muciniphila.*

According to a preferred embodiment, the invention relates to a synbiotic composition, comprising (i) a probiotic composition, wherein the probiotic composition comprises at least one *Roseburia* species; and (ii) a prebiotic mixture, wherein the prebiotic mixture comprises galacto-oligosaccharides (GOS) and inulin.

A preferred synbiotic composition according to the invention comprises a bacterial combination of *Roseburia* genus and *Akkermansia muciniphila,* as a probiotic, and inulin and GOS, as a prebiotic.

Preferred species of *Roseburia* include *Roseburia intestinalis, Roseburia cecicola, Roseburia faecis, Roseburia hominis* and *Roseburia inulinivorans.*

Culture conditions for *Akkermansia muciniphila* are disclosed, for example, in M. Derrien et al., lnt J Syst Evol, Microbiol. 2004 Sep; 54(Pt 5):1469-76. PubMed PMID: 15388697.

Culture conditions for *Roseburia intestinalis* are disclosed, for example, in SH Duncan et al., lnt J Syst Evol Microbiol. 2002 Sep; 52(Pt 5):1615-20. PubMed PMID: 12361264.

The invention also relates to a pharmaceutical composition comprising a symbiotic composition as disclosed above and a pharmaceutically acceptable vehicle.

Said symbiotic composition may be capsulated or lyophilised by freeze drying which is well known in the art.

The symbiotic composition can be a stable emulsion, where, in particular, the *Roseburia* genus and *Akkermansia muciniphila* are encapsulated with prebiotics in emulsion droplets.

Preferably, said pharmaceutical composition is in the form of enterically coated vegetable capsule, namely including an enteric coating when one or more of the probiotic organisms in the encapsulated formulation is non-viable under acidic conditions (e.g., the acidic conditions of the stomach).

In another embodiment, the synbiotic composition of the invention is a composition which is liquid at room temperature. In other embodiments, the pharmaceutical composition comprising a symbiotic composition is for example, in the form of tablets, capsules, powders, granules, solutions, emulsions, oral suspensions, drops, syrups, etc.. Advantageously, regardless of the galenic form, the pharmaceutical composition comprising a symbiotic composition is stable for at least one year when stored at temperatures between approximately 2 and 10 degrees Celsius.

A preferred pharmaceutical composition according to the invention comprises inulin and GOS blended with a specially selected bacterial combination of at least one species belonging to the *Roseburia* genus and *Akkermansia muciniphila.* In one embodiment, the final blend consists of about 2 billion colonies forming units (CFU)/capsule.

The invention further relates to a synbiotic composition as disclosed above for use in preventing and/or treating neurodegenerative disorders, in particular Alzheimer's disease or dementia.

The invention further relates to a method for preventing and/or treating neurodegenerative disorders, in particular Alzheimer's disease or dementia, comprising a step of administering the symbiotic composition as described above to a subject in need thereof.

When used for prevention and/or treatment of neurodegenerative diseases, such as Alzheimer's disease, a typical treatment regimen is one capsule, taken two times daily, but the dosage and regimen can be adjusted to the patient's needs.

The invention further relates to a synbiotic composition as disclosed above for use in the prevention and/or treatment of diseases or disorders related to pathological ageing in humans, such as, for example, obesity, diabetes, etc...

In yet another embodiment, the invention provides a method for carrying out a home-determination of the efficacy of a synbiotic composition as described above. Said method can comprise the steps of: providing instructions for administering a synbiotic composition over a predetermined time period to a subject and a questionnaire for the subject to self-record the effects of the administration of said symbiotic composition; collecting the self-recorded data from the questionnaire after the predetermined time period has passed; and evaluating the self-recorded data.

The questionnaire may be provided on-line.

Preferably, the pre-determined period is 1 month. Each individual will be provided with a score sheet query about the impact of the synbiotic composition on their overall health status. The query can be directed to both quantitative and qualitative parameters, such as, for example, their body weight, the meal they have ingested at the day, the intestinal comfort/discomfort feelings including nausea symptoms and appetite.

The invention further relates to a method for measuring the probability of a subject developping or having a neurodegenerative disorder, in particular Alzheimer's disease or dementia, comprising the steps of:
a) measuring the relative abundance of at least one *Roseburia* species in a sample obtained from a subject, and
b) determining the probability of the subject developing or having a neurodegenerative disorder based on the abundance measured in step a),
wherein a high relative abundance of at least one *Roseburia* species indicates a low probability of the subject developing or having a neurodegenerative disorder.

The sample can be a faecal sample. Preferably, the subject is human.

According to one embodiment, step a) consists of measuring the relative abundance of at least one *Roseburia* species and at least one *Verrucomicrobiales* species, wherein a high relative abundance of at least one *Roseburia* species and at least one *Verrucomicrobiales* species indicates a low probability of the subject developing or having a neurodegenerative disorder.

Preferred species of *Roseburia* include *Roseburia intestinalis, Roseburia cecicola, Roseburia faecis, Roseburia hominis* and *Roseburia inulinivorans.*

A preferred species of *Verrumicrobiales* is *Akkermansia muciniphila.*

### BRIEF DESCRIPTION OF THE DRAWINGS

Figure 1 describes the impact of the synbiotic composition of the invention on the level of cerebral Aβ42 (Figure 1A) and on cerebral plaques (Figure 1 B). Aβ42 is a pathogenic peptide involved in Alzheimer's disease as the main component of the amyloid plaques and on cerebral Aβ plaques in APPPS1 mice, a mouse model of Alzheimer.
Figure 2 describes the dose-dependent effect of the synbiotic composition of the invention on the level of cerebral Aβ42 in APPPS1 mice.

The invention is illustrated by the following examples.

### Example 1: Synbiotic composition

The ingredients of the synbiotic composition are shown in Table 1.

**Table 1**

| Ingredients | Weight % | Mg/capsule |
|---|---|---|
| **Probiotic mix** | 50 | 250 |
| *Akkermancia muciphiniphila* | | 125 |
| *Roseburia intestinalis* | | 125 |
| **Prebiotic mix** | 50 | 250 |
| Galacto-oligosaccharides (GOS) | 25 | 125 |
| Inulin | 25 | 125 |
| TOTAL | 100 | 500 |

Total CFU/capsule : 2 billion (2x 10⁹) CFU

The ingredients are in powder form.
Capsule composition: Maltodextrin, magnesium stearate, hydroxypropylmethyl cellulose, methacrylic acid, methyl methacrylate copolymer.

### Example 2: Efficacy of a synbiotic composition in an Alzheimer mouse model

Preclinical demonstration of the efficacy of a synbiotic composition containing *Roseburia intestinalis* and *Akkermansia muciniphila* as probiotic mix, and galacto-oligosaccharides (GOS) and inulin as prebiotic mix was performed as shown below by dietary intervention in an Alzheimer mouse model by selective modulation of gastro-intestinal microbiota and positive impact on Abeta amyloidosis (n= 5/group). Data are shown on Figure 1 as mean ± SEM Statistical differences between control and synbiotic composition-treated mice **:p<0.01;

The dose-dependent-effect is shown in Figure 2.

### a) APPPS1 mice

APPPS1 transgenic mice (Radde R. et al., EMBO Rep. 2006, (9):940-6) were maintained at animal core specific pathogen free facility. There was unlimited access to autoclaved food and water. APPPS1 animals co-express the KM670/671NL Swedish mutation of human amyloid precursor protein (APP) and the L166P mutation of human presenilin 1 (PS1) under the control of the Thy-1 promoter, and show age-dependent accumulation of parenchymal Aβ plaques with minimal vascular Aβ amyloid that is restricted to the pial vessels14. APPPS1 mice were generated on a C57BL/6 background. Both male and female APPPS1 mice as well as age-matched control wild-type (WT) littermates were used. APPPS1 mice and wildtype littermate were housed together in grouped cages (n = 5 mice/cage) until analyzed. Throughout the study these mice are referred to as conventionally-raised (CONVR) APPPS1 and WT mice.

### b) Preparation of the symbiotic composition

***Akkermansia muciniphila BAA35*** was grown in anaerobic condition using the following protocol and medium:
At Day 1, glass bottles used for liquid cultures were placed in the oven (3h at 180°C or 120°C overnight). Then the glassware were put under an anaerobic tent for at least 2 days prior being used.
At Day 3, media were split into anaerobic glass bottles (10ml in each) sealed with a rubber lid. At this stage, media were taken out of the anaerobic tent and placed them overnight at 37°C in order and check their sterility condition.
At Day 4, media can be used to culture bacteria or can be stored in the dark at 4°C.

The Anaerobic Akkermansia-Media is made of 18,5 g/l Brain Heart Infusion, 15 g/l Trypticase soy broth, 5 g/l yeast extract, 2,5 g/l K2HPO4, 1 µg/ml Hemin and 0,5 g/l Glucose. All reagents were mixed via sterile filtration prior to autoclave. The following compound were autoclaved separately: 0,4 g/l Na2CO3 autoclave, 0,5 g/l Cystein hydrochloride, 0,5 µg/ml Menadione filter sterile, 3 % FCS (complement-inactivated*) filter sterile 0,025 % Mucin (gastric mucin from porcine stomach,) separately 2 ml of 2.5% in water (100x) .The medium was pre-reduced in anaerobic tent for 2 days prior to be aliquoted

***Roseburia intestinalis L82*** is an aerobic strain and it grows in RUMEN BACTERIA MEDIUM made of Mineral solution. This buffers contains 0.30 g K₂HPO₄, 2.00 g Trypticase peptone (BD BBL), 0.50 g Yeast extract (OXOID), 3.10 ml Volatile fatty acid mixture, Haemin solution (0.05% w/v, see medium 104), 0.50 g Glycerol, Naresazurin solution (0.1% w/v), 4.00 g Na₂CO₃, D-Glucose 0.50 g, Maltose 0.50 g, Cellobiose 0.50 g, Starch, soluble 0.50 g, L-Cysteine-HCl x H₂O 0.25 g, Na₂S x 9 H₂O 0.25 g and distilled water was added qsp 1000ml.

Except glucose, maltose, cellobiose, soluble starch, cysteine and sulfide, all ingredients were dissolved in carbonate then inoculated to medium with 100% CO₂ gas for 30 - 45 min to make it anoxic. Carbonate was added and the medium was equilibrated with the CO₂ gas to pH 6.8. The medium was distributed under 100% CO₂ gas atmosphere into an anoxic Hungate-type tubes or serum vials prior to autoclave. Thereafter, glucose, maltose, cellobiose, soluble starch, cysteine and sulfide were from sterile anoxic stock solutions prepared under 100% N₂ gas atmosphere. Finally pH of complete medium was adjusted to 6.7 - 6.8. To complete the buffers, a mineral solution was prepared as follows: 12.00 g of KH₂PO₄ NaCl (NH₄)2SO₄ were mixed with 1.60 g CaCl₂ x 2 H₂O and 2.50 g MgSO₄ x 7 H₂O and filled up with distilled water 1000.00 ml. As an initial mimicking of the gastro-intestinal fermentative part, a volatile fatty acid mixture made of: 548.50 ml pure Acetic acid, 193.50 ml Propionic acid Butyric acid, 32.25 ml n-Valeric acid, 32.25 ml, 32.25ml isoButyric acid and 32.25 ml DL-2-Methyl butyric acid iso-Valeric acid was added.

After having cultured the bacteria separately, they have been washed twice with PBS 1X (4500 rpm for 10 min/each time) and at the end pooled in 1:1 ratio. 25 mg of Galactooligosaccharides (GOS) and 25mg of inulin powders were mixed to the pooled probiotics. The combination of the aforementioned bacteria and fibers constitutes the symbiotic composition. The procedure has been performed under axenic conditions in order to avoid external unwanted contaminations. Furthermore, the cleanness of the gavage inoculum has been checked by 16S rRNA sequencing on the left over from the gavage inoculum.

### c) Inoculation protocol

The synbiotic composition obtained as described above was administered to APPPS1 mice by oral gavage at 2×10¹¹ colony forming units per ml (For the single dose experiment described in Figure 1). 200 microliters were administered to the animals once or three times a day for 3 consecutive months. For dose dependent effect of the synbiotic composition , 2×10¹⁰ and 2×10¹¹ cfu/ml were administered to APPPS1 mice once or three times a day during 3 consecutive months.

### d) ELISA

Brain protein extracts and plasma were diluted to the fourth into sampling medium provided by the manufacturer and a final of 25 µl volume was loaded into a 96 well plates. Aβ38, Aβ40 and Aβ42 measurements were performed according to the manufacturer's instructions (Peptide Panel 1 (6E10) Kit (1 Plate) V-PLEX™K15200E-1 Mesoscale Discovery, Gaithersburg). All levels of Aβ38, Aβ40 and Aβ42 were normalized against total protein amount. For cytokine measurements, total brain homogenates were centrifuged at 14'000 rpm, 30 min, 4°C and supernatants were analyzed using the mouse pro-inflammatory panel 1 V-plex plate (Mesoscale Discovery) according to the manufacturer's instructions and normalized against total protein content.

### e) Quantification of Iba-1-positive immunoreactivity

Neocortical microglia immunoreactivity was quantified in hemi-brain sections immunostained for activated microglia (with the lba-1 antibody). Acquisition of images from Iba-1-stained sections was similar to that described for ThT quantification. Subsequently, a threshold of 110 with a variance of 10% was applied to each image using Image J. This threshold was selected in order to best display the microglia while minimizing the background noise. After visual inspection, ROI were selected such that staining artefacts on the section was manually removed by cropping. The microglia were quantified using the analysis particles tool embedded in ImageJ. In order to consider both the single microglia and the microglial loads, the pixel size was not specified and the circularity parameter was set to 0.0-1.0.

### f) Statistical analysis (Histology)

Data represent the means ± standard errors of the means. Statistical analysis was performed using ANOVA followed by Hom-Sidak's test for multiple comparisons and unpaired t test followed by Welch's correction.

### g) Statistical analysis (ELISA)

Graphpad Prism 6 software was used to perform statistical analysis. Data represent the means ± standard errors of the means. Statistical analysis was performed using ANOVA followed by Hom-Sidak's test for multiple comparisons.

### h) Results

The results show that a single dose of the synbiotic composition of the invention, when administered during 3 months to APPPS1 mice, either young or aged, decreases the levels of cerebral soluble Abeta and reduces the number of cerebral amyloid plaques (see Figure 1).

Furthermore, the synbiotic composition of the invention reduces cerebral soluble Abeta 42 in a dose-dependent manner when administered for 3 consecutive months.

## Claims

1. A symbiotic composition, comprising
i) a probiotic composition, which comprises at least one *Roseburia* species, and
ii) a prebiotic mixture, which comprises at least one dietary fiber.

2. The composition of claim 1, wherein the probiotic composition comprises at least one *Roseburia* species and at least one or more additional microbial species.

3. The composition of claim 1 or 2, wherein the probiotic composition comprises at least one *Roseburia* species and at least one *Verrucomicrobiales* species.

4. The composition according to any one of claims 1 to 3, wherein the at least one *Roseburia* species is selected from *Roseburia intestinalis, Roseburia cecicola, Roseburia faecis, Roseburia hominis* and *Roseburia inulinivorans.*

5. The composition according to any one of claims 2 to 4, wherein the at least one *Verrucomicrobiales* species is *Akkermansia muciniphila.*

6. The composition according to any one of claims 1 to 5, wherein said prebiotic mixture comprises at least one dietary fiber selected from non-starch derived indigestible polysaccharides, galacto-oligosaccharides and fructo-oligosaccharides, or mixtures thereof.

7. The composition according to any one of claims 1 to 6, wherein said prebiotic mixture comprises galacto-oligosaccharides (GOS) and inulin.

8. The composition of any one of claims 1 to 7, wherein the amount of prebiotic mixture is between 10% and 50% w/w.

9. The composition of any one of claims 1 to 8, which is in a dosage form containing 10⁶ to 10¹¹ viable colony forming units (CFU) per dose.

10. The composition of any one of claims 1 to 9, wherein the composition comprises at least 20% probiotics on a weight-percentage basis, preferably at least 40% probiotics on a weight-percentage basis.

11. The composition of any one of claims 1 to 10, comprising
i) a probiotic composition comprising at least one species of *Roseburia* genus and *Akkermansia muciniphila*, and
ii) a prebiotic mixture, comprising galacto-oligosaccarides(GOS) and inulin.

12. The composition of any one of claims 1 to 11, wherein the probiotic composition consists of 30wt% *Roseburia intestinalis,* 20wt% *Roseburia cecicola,* and 50wt% *Akkermansia muciniphila.*

13. A pharmaceutical composition containing a symbiotic composition according to any one of claims 1 to 12.

14. A synbiotic composition according to any one of claims 1 to 12, for use in preventing and/or treating neurodegenerative disorders, in particular Alzheimer's disease or dementia.

15. A method for carrying out a home-determination of the efficacy of a synbiotic composition according to any one of claims 1 to 12, comprising the steps of:
providing instructions for administering said synbiotic composition over a predetermined time period and a questionnaire for the subject to self-record the effects of the administration of said symbiotic composition; collecting the self-recorded data from the questionnaire after the predetermined time period has passed; and evaluating the self-recorded data.

16. A method for measuring the probability of a subject developping or having a neurodegenerative disorder, in particular Alzheimer's disease or dementia, comprising the steps of:
a) measuring the relative abundance of at least one *Roseburia* species in a sample obtained from a subject, and
b) determining the probability of the subject developing or having a neurodegenerative disorder based on the abundance measured in step a),
wherein a high relative abundance of at least one *Roseburia* species indicates a low probability of the subject developing or having a neurodegenerative disorder.
